# FASCICULE DE BREVET EUROPEEN

(11) **EP 3 641 712 B1**
(45) Date de publication et mention de la délivrance du brevet: **20.09.2023**
(21) Numéro de dépôt: 18748994.3
(22) Date de dépôt: 25.06.2018
(51) Int. Cl.: A61F 13/14, A61F 13/15

(54) **COUSSINET D'ALLAITEMENT LAVABLE MULTICOUCHE**
WASCHBARE MEHRSCHICHTIGE STILLEINLAGE
WASHABLE MULTILAYER NURSING PAD

(30) Priorité: 23.06.2017 FR 1755791
(43) Date de publication de la demande: 29.04.2020
(73) Titulaire: Cache-Coeur, 29480 Le Relecq Kerhuon (FR)
(72) Inventeur: TROLLIET, Audrey, 29200 Brest (FR)
(74) Mandataire: Ipside
(86) Numéro de dépôt international: PCT/FR2018/051531
(87) Numéro de publication internationale: WO 2018/234719

(56) Documents cités:
- WO-A1-2011/133106
- WO-A2-01/34080
- GB-A- 2 406 796
- US-A- 3 442 268

## Description

### DOMAINE TECHNIQUE DE L'INVENTION

La présente invention concerne un coussinet d'allaitement lavable multicouche. Elle s'applique, en particulier, au domaine de la lingerie pour futures mamans ou mamans allaitantes ayant la nécessité d'absorber des pertes de lait.

### ETAT DE LA TECHNIQUE

Il est connu deux types de coussinets : jetable et lavable. Un coussinet d'allaitement lavable est un coussinet réutilisable et durable contrairement aux coussinets d'allaitement jetables.

Les coussinets jetables peuvent causer des irritations à cause du film plastique utilisé pour l'étanchéité qui fait barrière à la circulation de l'air. De plus les coussinets jetables sont composés de substances toxiques et leur usage est unique.

Les coussinets lavables existants n'isolent pas la peau de l'humidité, ce qui cause des irritations et fait coller les matières à la peau.

Les coussinets lavables sont plats, ceux qui ne le sont pas prennent la forme avec une pince qui crée une fuite due aux trous d'aiguilles. Enfin, les coussinets lavables offrent une capacité d'absorption moyenne de 20 ml de lait par coussinet et les coussinets jetables offrent une absorption maximale de 75 ml.

Certains documents de l'art antérieur proposent également des dispositifs de coussinets d'allaitement lavables, comme par exemple, le document WO2011/133106 qui décrit des coussinets d'allaitement lavables ayant des membranes microporeuses et le document WO 01/34080 qui décrit des couches de maille tricotées ayant des propriétés de perméabilité.

On connaît par exemple le document de publication WO2005034824 dans lequel est décrit un coussinet qui comprend trois couches scellées ensemble à leur périphérie par soudage par ultrasons. Toutefois, ce type de coussinet n'est pas satisfaisant car il n'isole pas la peau de l'humidité et n'est pas antibactérien. Ce coussinet n'est pas satisfaisant car les matières sont libres et la couche intérieure peut coller à la peau humide. Ce coussinet n'est pas satisfaisant car il n'est pas ergonomique et sa forme ne s'adapte pas aux différentes formes de soutien-gorge.

### OBJET DE L'INVENTION

La présente invention vise à remédier à ces inconvénients.

A cet effet, selon un premier aspect, la présente invention vise un coussinet d'allaitement lavable multicouche, remarquable en ce qu'il comporte un bord et des couches successives :
- une couche de maille tricotée d'au moins 1,5 mm d'épaisseur selon la norme ISO 5084 et perforée, comportant une face supérieure en contact direct avec la peau et une face inférieure dont :
   - la face supérieure est composée d'une première couche de fibre textile en contact direct avec la peau, ladite première couche de fibre textile est composée d'une pluralité d'ouvertures perforantes sur une surface externe,
   - la face inférieure est composée d'une deuxième couche de fibre textile en contact avec une couche de fibres non tissées,
   - la première couche de fibre textile est en polyester antibactérien et la deuxième couche de fibre est en polyester,
   - une couche mono-filament d'au moins 1,5 mm d'épaisseur selon la norme ISO 5084 composée de fibre textile, insérée entre la première couche de fibre textile (24) et la deuxième couche de fibre textile ;
- la couche de fibres non tissées, en contact avec la face supérieure adaptée à absorber au moins 2,5 L/m2 d'eau ;
- une couche microporeuse comportant une structure imperméable et respirante ;
- une couche de maille protectrice recouvrant toute la couche microporeuse.

La couche de maille tricotée est en trois dimensions (3D) et permet la circulation de l'air et de maintenir la peau au sec en l'isolant de l'humidité. La couche de maille 3D tricotée permet d'éviter la prolifération de bactéries. La couche de maille tricotée 3D permet d'éviter toute odeur.

La couche de maille tricotée comporte un coeur aéré.

Concernant l'épaisseur de 1,5 mm. La mesure de cette épaisseur est effectuée selon la norme ISO 5084. Le mesureur d'épaisseur adopte le principe de détermination de l'épaisseur d'un textile qui consiste à mesurer la distance perpendiculairement entre deux plaques de référence, ici entre la face supérieure et face inférieure de la couche de maille tricotée, appliquant sur le textile une pression de 0,1 kPa ou de 1 kPa.

L'enchevêtrement des fibres non tissées est mécanique. La couche de fibres non tissées est réalisée à l'aide d'aiguilles qui transforment les fibres en une matière non tissée. La matière composant la couche de fibres non tissées bloque le liquide après absorption. La couche de fibres non tissées est super absorbante.

Les coussinets d'allaitement lavables de l'art antérieur offrent une absorption moyenne de 20 ml de lait par coussinet. Les coussinets d'allaitement jetables offrent une absorption moyenne de 75 ml de lait. La couche de fibres non tissées donne au coussinet lavable tel que décrit, une capacité d'absorption au moins égale aux coussinets d'allaitement jetables. La capacité d'absorption est d'au moins 65 ml de lait, allant jusqu'à 80ml de lait.

La membrane constitue une barrière au liquide tout en laissant l'air circuler vers la peau en accélérant le séchage. La membrane microporeuse permet d'éviter les fuites de lait. La membrane est recouverte d'une couche de maille protectrice.

L'invention est avantageusement mise en oeuvre selon les modes de réalisation et les variantes exposées ci-après, lesquelles sont à considérer individuellement ou selon toute combinaison techniquement opérante.

On entend par fibre textile, une fibre naturelle, synthétique ou artificielle.

Dans un mode de réalisation, la première couche de fibre textile est en polyester antibactérien et la deuxième couche de fibre est en polyester.

Selon les deux modes de réalisation précédents, la couche de maille tricotée est composée de trois couches en trois matières différentes et comprend :
- la face supérieure composée d'une couche polyester antibactérien, ladite couche polyester antibactérien est composée d'une pluralité d'ouvertures perforantes sur une surface externe ;
- la face inférieure composée d'une couche polyester en contact avec la couche de fibres non tissées ;
- une couche mono-filament d'au moins 1,5 mm composée de polyester, tricotée entre la couche polyester antibactérien et la couche polyester.

La fibre antibactérienne permet d'éviter toute prolifération de bactéries due aux protéines de lait pour une hygiène garantie.

La structure perforée permet le drainage du lait. Les perforations ont une ouverture d'une taille de l'ordre de la grosseur d'une aiguille.

La structure 3D très aérée de la couche mono-filament permet une circulation de l'air. La structure très aérée de la couche mono-filament isole la peau de l'humidité. La structure de la couche mono-filament diminue fortement le risque d'irritation.

Dans un mode de réalisation, la couche de fibres non tissées a une épaisseur comprise entre 2,5 mm et 8 mm, de préférence entre 5 mm et 7 mm.

Plus l'épaisseur de la couche de fibres non tissées est grande plus la capacité d'absorption du coussinet est élevée.

Dans un mode de réalisation, le coussinet comporte une forme triangulaire avec les angles arrondis.

La forme triangulaire permet de rendre le coussinet invisible sous les vêtements. La forme triangulaire s'adapte à toutes les formes de soutien gorge.

Dans un mode de réalisation, le coussinet a deux tailles permettant de varier la capacité d'absorption selon les flux et de s'adapter aux différents volumes de poitrines.

Dans un mode de réalisation, le coussinet comporte une préformation bombée pour épouser le galbe du sein.

La préformation pour épouser la forme du sein permet d'éviter un sentiment d'inconfort et d'améliorer la discrétion.

Dans un mode de réalisation, le coussinet comporte un bord soudé.

On entend par bord soudé : un bord découpé à la soudure ou à la découpe aux ultrasons. L'effet technique de la soudure permet de rendre étanche le bord du coussinet. La soudure ou la coupe par ultrasons permet d'éviter les piqûres dans le tissu et améliore l'imperméabilité ainsi que les propriétés isolante du textile. Il évite aussi l'effilochage car les fibres sont fondues au niveau de la découpe. Le procédé de découpe est très rapide.

Dans un mode de réalisation, le coussinet comporte une préformation bombée ayant une cavité sphérique positionnée sensiblement au centre du coussinet sur la face interne.

Les pointes de sein sont rendues très sensibles par l'allaitement. La préformation permet d'éviter les irritations et les frottements.

Dans un mode de réalisation, le coussinet comporte un bordage formant un retour sur le bord du coussinet.

Le bordage permet de rendre le coussinet étanche sur la tranche en évitant les fuites de lait par les bords. Le bordage est contrecollé, évitant les risques de fuites de lait par les trous d'aiguilles. Le bordage est doté d'un traitement antidérapant permettant d'éviter au coussinet de se déplacer dans le bonnet du soutien gorge.

Dans un mode de réalisation, le bordage est composé d'une bandelette recouvrant une partie de la face supérieure et de la face inférieure du coussinet.

Dans un mode de réalisation, le bordage comporte un revêtement, de type silicone ou polyuréthane, pour appliquer un effet antidérapant.

Dans un mode de réalisation, le coussinet comporte un revêtement, de type silicone ou polyuréthane. Dans un exemple, le revêtement est un motif appliqué sur le coussinet.

Selon un deuxième aspect, la présente invention vise un procédé de fabrication d'un coussinet d'allaitement lavable multicouche, il comporte :
- a) une étape de contre collage à partir des couches successives suivantes :
   ▪ une couche de maille tricotée,
   ▪ une couche de fibres non tissées,
   ▪ une couche microporeuse, et
   ▪ une couche protectrice,
   ladite étape de contre collage forme un ensemble multicouche.

Dans un mode de réalisation, le procédé comporte également l'étape suivante :
- b) une étape de préformage du coussinet à partir de l'ensemble multicouche formé à l'étape a), ladite étape de préformage est configurée pour bomber l'ensemble multicouche ;

Dans un mode de réalisation, le procédé comporte une étape suivante, après l'étape b) : une étape c) de soudure du bord du coussinet.

Dans un mode de réalisation, comportant également une étape suivante, après l'étape c) : une étape d) une étape de bordage par thermocollage du coussinet formant une surépaisseur sur le bord du coussinet.

L'étape de contre collage permet de respecter les caractéristiques techniques des matières et de préserver la respirabilité du coussinet. Le contre collage permet d'assembler les quatre couches en une seule.

L'étape de préformage donne au coussinet la forme du sein et la cavité pour la préformation du mamelon.

La finition réalisée à cette étape donne au coussinet la possibilité d'isoler le lait et éviter toute fuite éventuelle par les bords mais également la finition des bords.

Les avantages, buts et caractéristiques particuliers de ce procédé de fabrication d'un coussinet d'allaitement lavable étant similaires à ceux du coussinet d'allaitement lavable multicouche objet de la présente invention, ils ne sont pas rappelés ici.

### BREVE DESCRIPTION DES FIGURES

D'autres avantages, buts et caractéristiques de la présente invention ressortent de la description qui suit, faite, dans un but explicatif et nullement limitatif, en regard des dessins annexés, dans lesquels :
- la figure 1, représente, une vue éclatée d'un mode de réalisation du coussinet comportant quatre couches du coussinet d'allaitement.
- la figure 2 représente, une vue extérieure du coussinet.
- la figure 3 représente, une vue intérieure du coussinet.
- la figure 4 représente, une vue latérale du coussinet.
- la figure 5 représente, une vue latérale schématique du coussinet en coupe selon un axe longitudinal A-A.
- la figure 6 représente, les étapes de fabrication du coussinet.

### DESCRIPTION D'EXEMPLES DE REALISATION DE L'INVENTION

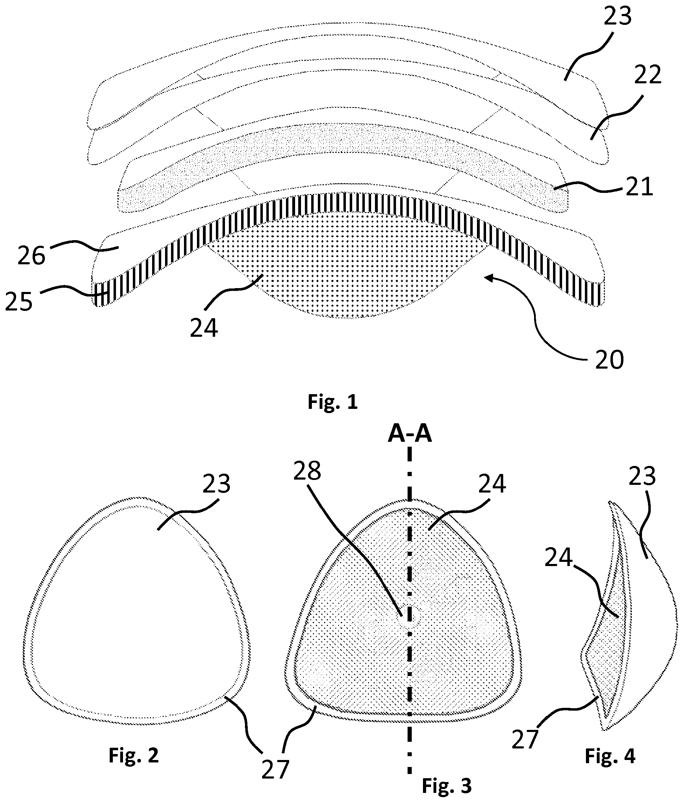

Dans un mode de réalisation le coussinet d'allaitement lavable comporte trois couches successives. La figure 1 représente un autre mode de réalisation dans lequel le coussinet d'allaitement comporte quatre couches successives. La figure 1 représente une vue éclatée des quatre couches du coussinet d'allaitement.

La figure 1 comporte une couche de maille tricotée 20 3D, une couche de fibres non tissées 21, une couche microporeuse 22, une couche protectrice 23, une couche polyester antibactérien 24, une couche mono-filament 25 et une couche polyester 26.

La première couche est une couche de maille tricotée 20 3D réalisée en trois dimensions par un tricotage de trois matières différentes. La couche de maille tricotée 20 3D comporte d'une face inférieure comportant une couche polyester antibactérien 24. La couche polyester antibactérien 24 est en contact direct avec la peau. La structure de la couche polyester antibactérien 24 est perforée. La structure perforée de la couche polyester antibactérien 24 laisse évacuer le lait dans les couches supérieures. Dans un mode de réalisation, la couche polyester antibactérien 24 a une masse d'au moins 75 g/m2. Dans un autre mode de réalisation, la couche polyester antibactérien 24 a une masse de 100g/m2. La couche de polyester antibactérien, pour obtenir sa caractéristique antibactérienne subit un traitement spécifique. Il existe différents types de traitements antibactériens pour les textiles. Par exemple, il est possible d'utiliser la technique d'apprêtage dans laquelle le textile est plongé dans une solution comportant un principe actif et un liant. Le liant permet au principe actif de rester collé au textile. La maille tricotée 20 3D est composée d'une face supérieure comportant une couche polyester 26. La structure de la couche polyester 26 est structurée de manière à transférer le liquide vers les couches supérieures. Dans un mode de réalisation, la couche polyester 26 a une masse d'au moins 100 g/m2. Dans un autre mode de réalisation, la couche polyester 26 a une masse de 135 g/m2. Le coeur de la couche de maille tricotée 20 est composée d'une couche mono-filament 25 en polyester. Un mono filament et un filament textile unique de grande longueur, obtenu dans la fabrication des textiles artificiels ou synthétiques donnant à ce textile une structure très aérée. Dans un mode de réalisation, la couche mono-filament 25 a une masse d'au moins 70 g/m2. Dans un autre mode de réalisation, la couche mono-filament 25 a une masse de 95 g. La couche mono-filament 25 est tricotée entre la couche polyester antibactérien 24 et la couche polyester 26.

La deuxième couche est une couche de fibres non tissées 21 positionnée au dessus de la face supérieure de la couche de maille tricotée 20 3D. La couche de fibres non tissées 21 est composée par exemple de 50% de polyester et 50% de viscose. La couche de fibres non tissées 21 est obtenue par transformation des fibres à l'aide d'un système mécanique à aiguilles. La densité de la couche de fibres non tissées 21 est d'au moins 75 g/mm d'épaisseur. Dans un mode de réalisation la densité de la couche de fibres non tissées 21 est de 100g/mm d'épaisseur. La couche de fibres non tissées 21 absorbe par exemple au moins 5 L/m² de lait et au moins 8 L/m² d'eau. Dans un mode de réalisation la couche de fibres non tissées 21 absorbe 6,45 L/m² de lait et 9,27 L/m² d'eau. La couche de fibres non tissées 21 se positionne au dessus de la couche de maille tricotée 20 3D.

La troisième couche est une couche microporeuse 22. La couche microporeuse 22 est imperméable et respirante. La couche microporeuse 22 est composée dans un mode de réalisation à 60% de polyester et à 40% de polyuréthane. Dans un autre mode de réalisation, la couche microporeuse est composée à 100% de polyester. La structure de la couche microporeuse 22 lui donne la caractéristique de respirabilité tout en retenant le lait. La couche microporeuse 22 donne au coussinet d'allaitement sa caractéristique d'imperméabilité. La couche microporeuse 22 se positionne au dessus de la couche de fibres non tissées 21.

La quatrième couche est une couche protectrice 23. La structure du voile protecteur est légère et aérée tout en conférant à la couche protectrice 23 la caractéristique d'éviter les accros et les griffures. La couche protectrice 23 se positionne au dessus de la couche microporeuse 22. La couche protectrice 23 est située à l'opposé de la couche de maille tricotée 20 3D.

La figure 2 représente, une vue extérieure du coussinet. La figure 2 comporte la couche protectrice 23 et le bordage 27 par une bandelette thermocollée. Le coussinet a une forme de triangle équilatéral. Le coussinet a une forme comportant des angles arrondis. Les bords du coussinet sont légèrement arrondis.

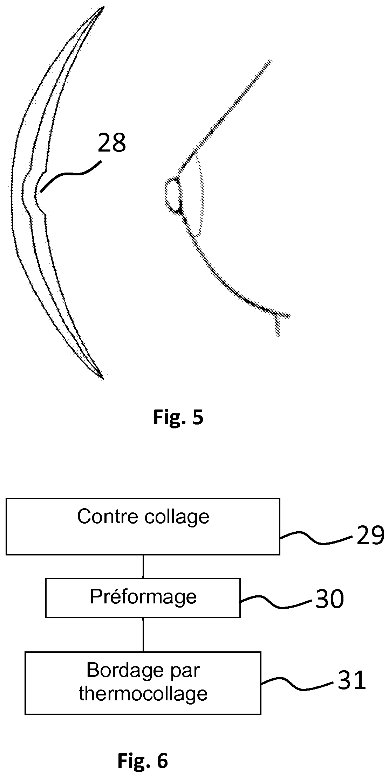

La figure 3 représente une vue intérieure du coussinet. La figure 3 comporte la couche polyester antibactérien 24, le bordage 27 par une bandelette thermocollée et la cavité du mamelon préformé 28. Le renfoncement du mamelon préformé 28 est réalisé pour diminuer les sensations d'irritation et d'inconfort. La figure 3 présente un axe longitudinal A-A utilisé par la suite pour la figure 5.

La figure 4 représente une vue latérale du coussinet. La figure 4 représente comporte la couche polyester antibactérien 24, la couche protectrice 23 et le bordage 27 par une bandelette thermocollée. Le coussinet d'allaitement est courbé de manière à épouser le galbe du sein.

La figure 5 représente une vue latérale schématique du coussinet en coupe longitudinale selon un axe A-A. La figure 5 comporte la couche de fibres non tissées 21, la couche de maille tricotée 3D 20 et la cavité du mamelon préformé 28. La profondeur du renfoncement du mamelon préformé 28 est entre 0.1 et 0.5 cm. Dans un mode de réalisation, la profondeur du renfoncement du mamelon préformé 28 est de 0,3 cm. Dans sa partie la plus épaisse, le coussinet a une épaisseur entre 0.2 et 0.9 cm. Dans un mode de réalisation, l'épaisseur du coussinet est de 0,8 cm. Le diamètre du renfoncement du mamelon préformé 28 est d'au moins 1 cm. Dans un mode de réalisation, le diamètre du renfoncement du mamelon préformé 28 est de 1,5 cm.

La figure 6 représente un mode de réalisation du coussinet d'allaitement lavable.

La première étape consiste au contre collage 29. Le contre collage 29 est un assemblage de matériaux par procédés de collage. Dans un mode de réalisation, le procédé de contre collage 29 est réalisé à partir des couches successives suivantes :
▪ une couche de maille tricotée (20),
▪ une couche de fibres non tissées (21),
▪ une couche microporeuse (22), et
▪ une couche protectrice (23).

Le procédé de contre collage 29 forme un ensemble multicouche. Le procédé de contre collage 29 respecte les caractéristiques techniques des différentes matières composant les couches successives ainsi que la respirabilité du coussinet.

La deuxième étape consiste en un préformage 30 du coussinet. Le préformage 30 est réalisé à partir de l'ensemble multicouche formé par le procédé de contre collage 29. Le préformage 30 consiste à donner au coussinet une forme bombée épousant le galbe du sein. Le préformage 30 donne une forme bombée ayant une cavité sphérique positionné sensiblement au centre du coussinet sur la face interne créant un renfoncement pour le mamelon.

La troisième étape consiste en un bordage par thermocollage 31. Le bordage par thermocollage 31 est la pour former le bord du coussinet. Le bord est définit par une tranche et une partie de la face supérieure et une partie de la face inférieure du coussinet. Le bord du coussinet est matérialisé par une bandelette. Le bordage 27 se compose d'une bandelette d'au moins 7 mm qui recouvre par 3 mm chaque coté du coussinet et par 1 mm la tranche du coussinet. Dans un mode de réalisation le bordage 27 comporte une bandelette de 16mm qui recouvre par 7 mm chaque coté du coussinet et par 2 mm la tranche du coussinet. La bandelette utilisée pour le thermocollage bénéficie d'une finition antidérapante.

### NOMENCLATURE

- 20: Couche de maille tricotée
- 21: Couche de fibres non tissées
- 22: Couche microporeuse
- 23: Couche protectrice
- 24: Couche polyester antibactérien
- 25: Couche mono-filament
- 26: Couche polyester
- 27: Bordage
- 28: Renfoncement du mamelon préformé
- 29: Contre collage
- 30: Préformage
- 31: Bordage par thermocollage

## Revendications

1. Coussinet d'allaitement lavable multicouche, **caractérisé en ce qu'**il comporte un bord et des couches successives :
- une couche de maille tricotée (20) d'au moins 1,5 mm d'épaisseur selon la norme ISO 5084 et perforée, comportant une face supérieure en contact direct avec la peau et une face inférieure dont :
- la face supérieure est composée d'une première couche de fibre textile (24) en contact direct avec la peau, ladite première couche de fibre textile (24) est composée d'une pluralité d'ouvertures perforantes sur une surface externe,
- la face inférieure est composée d'une deuxième couche de fibre textile (26) en contact avec une couche de fibres non tissées (21),
- la première couche de fibre textile (24) est en polyester antibactérien et la deuxième couche de fibre (26) est en polyester,
- une couche mono-filament (25) d'au moins 1,5 mm d'épaisseur selon la norme ISO 5084 composée de fibre textile, insérée entre la première couche de fibre textile (24) et la deuxième couche de fibre textile (26) ;
- la couche de fibres non tissées (21), en contact avec la face supérieure adaptée à absorber au moins 2,5 L/m² d'eau ;
- une couche microporeuse (22) comportant une structure imperméable et respirante ;
- une couche protectrice (23) recouvrant toute la couche microporeuse.

2. Coussinet selon la revendication 1, dans lequel la couche de fibres non tissées (21) a une épaisseur comprise entre 2,5 mm et 8 mm, de préférence entre 5 mm et 7 mm.

3. Coussinet selon la revendication 1, dans lequel le coussinet comporte une forme sensiblement triangulaire avec des angles arrondis.

4. Coussinet selon la revendication 1, dans lequel le coussinet comporte une préformation bombée ergonomique pour épouser le galbe du sein.

5. Coussinet selon la revendication 1, dans lequel le coussinet comporte un bord soudé.

6. Coussinet selon la revendication 1, dans lequel le coussinet comporte une préformation bombée ayant une cavité sphérique positionné sensiblement au centre du coussinet sur la face interne.

7. Coussinet selon la revendication 1, dans lequel le coussinet comporte un bordage (27) formant un retour sur le bord du coussinet, ledit bordage (27) est composé d'une bandelette étanche recouvrant une partie de la face supérieure et de la face inférieure du coussinet et formant une surépaisseur sur le bord du coussinet.

8. Coussinet selon la revendication 7, dans lequel le bordage (27) comporte un revêtement, de type silicone ou polyuréthane, pour appliquer un effet antidérapant.

9. Coussinet selon la revendication 1, dans lequel le coussinet comporte un revêtement, de type silicone ou polyuréthane.

10. Procédé de fabrication d'un coussinet d'allaitement lavable multicouche selon les revendications 1 à 9, **caractérisé en ce qu'**il comporte :
- a) une étape de contre collage (29) à partir des couches successives suivantes :
▪ une couche de maille tricotée (20),
▪ une couche de fibres non tissées (21),
▪ une couche microporeuse (22), et
▪ une couche protectrice (23),
ladite étape de contre collage (29) forme un ensemble multicouche.

11. Procédé selon la revendication 10 comportant également l'étape suivante, après l'étape a) :
- b) une étape de préformage (30) du coussinet à partir de l'ensemble multicouche formé à l'étape a), ladite étape de préformage (30) est configurée pour bomber l'ensemble multicouche.

12. Procédé selon la revendication 11 comportant également une étape suivante, après l'étape b) :
- c) une étape de soudure du bord du coussinet.

13. Procédé selon la revendication 12 comportant également une étape suivante, après l'étape c) :
- d) une étape de bordage par thermocollage (31) du coussinet formant une surépaisseur étanche sur le bord du coussinet.

## Patentansprüche

1. Waschbare mehrschichtige Stilleinlage, **dadurch gekennzeichnet, dass** sie einen Rand und aufeinanderfolgende Schichten umfasst:
- eine gewirkte und perforierte Maschenschicht (20) von mindestens 1,5 mm Dicke gemäß der Norm ISO 5084, die eine Oberseite in direktem Kontakt mit der Haut und eine Unterseite umfasst:
- deren Oberseite aus einer ersten Textilfaserschicht (24) in direktem Kontakt mit der Haut besteht, wobei die erste Textilfaserschicht (24) aus einer Vielzahl von Perforationsöffnungen an einer Außenfläche besteht,
- deren Unterseite aus einer zweiten Textilfaserschicht (26) in Kontakt mit einer Vliesfaserschicht (21) besteht,
- deren erste Textilfaserschicht (24) aus antibakteriellem Polyester ist und zweite Faserschicht (26) aus Polyester ist,
- eine aus Textilfaser bestehende Monofilamentschicht (25) von mindestens 1,5 mm Dicke gemäß der Norm ISO 5084, die zwischen der ersten Textilfaserschicht (24) und der zweiten Textilfaserschicht (26) eingefügt ist;
- wobei die mit der Oberseite in Kontakt stehende Vliesfaserschicht (21) geeignet ist, mindestens 2,5 L/m² Wasser zu absorbieren;
- eine mikroporöse Schicht (22), die eine undurchlässige und atmungsaktive Struktur umfasst;
- eine Schutzschicht (23), die die ganze mikroporöse Schicht bedeckt.

2. Einlage nach Anspruch 1, wobei die Vliesfaserschicht (21) eine Dicke im Bereich zwischen 2,5 mm und 8 mm, vorzugsweise zwischen 5 mm und 7 mm aufweist.

3. Einlage nach Anspruch 1, wobei die Einlage eine im Wesentlichen dreieckige Form mit abgerundeten Ecken umfasst.

4. Einlage nach Anspruch 1, wobei die Einlage eine ergonomisch gewölbte Vorformung umfasst, um sich der Rundung der Brust anzupassen.

5. Einlage nach Anspruch 1, wobei die Einlage einen verschweißten Rand umfasst.

6. Einlage nach Anspruch 1, wobei die Einlage eine gewölbte Vorformung umfasst, die einen kugelförmigen Hohlraum aufweist, der im Wesentlichen in der Mitte der Einlage auf der Innenseite positioniert ist.

7. Einlage nach Anspruch 1, wobei die Einlage eine Umrandung (27) umfasst, die einen Umschlag am Rand der Einlage bildet, wobei die Umrandung (27) aus einem dichten Streifen besteht, der einen Teil der Oberseite und der Unterseite der Einlage bedeckt und eine Verdickung am Rand der Einlage bildet.

8. Einlage nach Anspruch 7, wobei die Umrandung (27) eine Beschichtung vom Silikon- oder Polyurethan-Typ umfasst, um einen rutschhemmenden Effekt aufzubringen.

9. Einlage nach Anspruch 1, wobei die Einlage eine Beschichtung vom Silikon- oder Polyurethan-Typ umfasst.

10. Verfahren zur Herstellung einer waschbaren mehrschichtigen Stilleinlage nach den Ansprüchen 1 bis 9, **dadurch gekennzeichnet, dass** es umfasst:
- a) einen Schritt des Kaschierens (29) ausgehend von den folgenden aufeinanderfolgenden Schichten:
- eine gewirkte Maschenschicht (20),
- eine Vliesfaserschicht (21),
- eine mikroporöse Schicht (22), und
- eine Schutzschicht (23),
wobei der Schritt des Kaschierens (29) eine mehrschichtige Anordnung bildet.

11. Verfahren nach Anspruch 10, das nach Schritt a) ebenfalls den folgenden Schritt umfasst:
- b) einen Schritt des Vorformens (30) der Einlage ausgehend von der in Schritt a) gebildeten mehrschichtigen Anordnung, wobei der Schritt des Vorformens (30) so konfiguriert ist, dass die mehrschichtige Anordnung gewölbt wird.

12. Verfahren nach Anspruch 11, das nach Schritt b) ebenfalls einen folgenden Schritt umfasst:
- c) einen Schritt des Verschweißens des Randes der Einlage.

13. Verfahren nach Anspruch 12, das nach Schritt c) ebenfalls einen folgenden Schritt umfasst:
- d) einen Schritt des Umrandens durch Heißverkleben (31) der Einlage, der eine dichte Verdickung am Rand der Einlage bildet.

## Claims

1. Washable multilayer nursing pad, **characterised in that** it comprises an edge and successive layers:
- a knitted mesh layer (20) at least 1.5 mm thick according to the ISO 5084 standard and perforated, comprising a top face in direct contact with the skin and a bottom face of which;
- the top face is composed of a first textile fibre layer (24) in direct contact with the skin, said first textile fibre layer (24) is composed of a plurality of perforating openings on an outer surface;
- the bottom face is composed of a second textile fibre layer (26) in contact with the non-woven fibre layer (21);
- the first textile fibre layer (24) is made of antibacterial polyester and the second fibre layer (26) is made of polyester,
- a monofilament layer (25) at least 1.5 mm thick according to the ISO 5084 standard composed of textile fibre, inserted between the first textile fibre layer (24) and the second textile fibre layer (26).
- the non-woven fibre layer (21), in contact with the top face, capable of absorbing at least 2.5 L/m² of water;
- a microporous layer (22) comprising an impermeable and breathable structure;
- a protective layer (23) covering the entire microporous layer.

2. Pad according to claim 1, wherein the non-woven fibre layer (21) has a thickness between 2.5 mm and 8 mm, preferably between 5 mm and 7 mm.

3. Pad according to claim 1, wherein the pad comprises a substantially triangular shape with rounded angles.

4. Pad according to claim 1, wherein the pad comprises an ergonomic rounded preformation to mould the shape of the breast.

5. Pad according to claim 1, wherein the pad comprises a welded edge.

6. Pad according to claim 1, wherein the pad comprises a rounded preformation having a spherical cavity positioned substantially at the centre of the pad on the inner face.

7. Pad according to claim 1, wherein the pad comprises edging (27) forming a return on the edge of the pad, said edging (27) is composed of an impervious strip covering a part of the top face and of the bottom face of the pad and forming an extra thickness on the edge of the pad.

8. Pad according to claim 7, wherein the edging (27) comprises a coating, of silicone or polyurethane type, to apply a non-slip effect.

9. Pad according to claim 1, wherein the pad comprises a coating, of silicone or polyurethane type.

10. Method for manufacturing a washable multilayer nursing pad according to claims 1 to 9, **characterised in that** it comprises:
- a) a step of lamination (29) using the following successive layers:
▪ a knitted mesh layer (20),
▪ a non-woven fibre layer (21),
▪ a microporous layer (22), and
▪ a protective layer (23),
said step of lamination (29) forms a multilayer assembly.

11. Method according to claim 10 also comprising a subsequent step, after step a):
- b) a step of preforming (30) of the pad from the multilayer assembly formed in step a), said step of preforming (30) is configured to render the multilayer assembly convex.

12. Method according to claim 11 also comprising a subsequent step, after step b):
- c) a step of welding of the edge of the pad.

13. Method according to claim 12 also comprising a subsequent step, after step c):
- d) a step of edging by thermofusing (31) of the pad forming an impervious extra thickness on the edge of the pad.
